Europäisches Patentamt

European Patent Office

Office européen des brevets

(11) Veröffentlichungsnummer: **0 339 426 B1**

(12) EUROPÄISCHE PATENTSCHRIFT

(45) Veröffentlichungstag der Patentschrift: **14.09.94**

(21) Anmeldenummer: **89106841.3**

(22) Anmeldetag: **17.04.89**

(51) Int. Cl.5: **C07B 41/04**, C07C 43/11,
C07C 43/196, C07C 69/22,
C07C 213/00, C07C 41/03,
C07C 67/26

(54) **Verwendung von calcinierten Hydrotalciten als Katalysatoren für die Ethoxylierung bzw. Propoxylierung.**

(30) Priorität: **25.04.88 DE 3813910**
**23.12.88 DE 3843713**

(43) Veröffentlichungstag der Anmeldung:
**02.11.89 Patentblatt 89/44**

(45) Bekanntmachung des Hinweises auf die
Patenterteilung:
**14.09.94 Patentblatt 94/37**

(84) Benannte Vertragsstaaten:
**AT BE CH DE ES FR GB IT LI NL**

(56) Entgegenhaltungen:
**DE-A- 3 833 076**

**CHEMICAL ABSTRACTS, Band 95, Nr. 11, 14.
September 1981, Seite 590, Zusammenfassung Nr. 97099m, Columbus, Ohio, US; &
JP-A-81 36 431 (MITSUI TOATSU CHEMICALS,
INC.) 09-04-1981**

(73) Patentinhaber: **Henkel Kommanditgesellschaft
auf Aktien**

**D-40191 Düsseldorf (DE)**

(72) Erfinder: **Behler, Ansgar, Dr.
Siegfriedstrasse 80
D-4250 Bottrop (DE)**
Erfinder: **Endres, Helmut, Dr.
Benrodestrasse 82
D-4000 Düsseldorf 13 (DE)**
Erfinder: **Friedrich, Klaus, Dr.
Marconistrasse 13
D-4000 Düsseldorf 13 (DE)**
Erfinder: **Herrmann, Klaus
Köpenicker Strasse 33
D-4019 Monheim (DE)**

EP 0 339 426 B1

**Beschreibung**

Die Erfindung betrifft die Verwendung von calcinierten Hydrotalciten als Katalysatoren für die Ethoxylierung bzw. Propoxylierung von Verbindungen mit aktiven H-Atomen.

Hydrotalcit ist ein natürliches Mineral mit der Idealformel

$$Mg_6Al_2(OH)_{16}CO_3.4H_2O$$

dessen Struktur von derjenigen des Brucits ($Mg(OH)_2$) abgeleitet ist. Brucit kristallisiert in einer Schichtstruktur mit den Metallionen in Oktaederlücken zwischen zwei Schichten aus dichtgepackten Hydroxylionen, wobei nur jede zweite Schicht der Oktaederlücken besetzt ist. Im Hydrotalcit sind einige Magnesiumionen durch Aluminiumionen ersetzt, wodurch das Schichtpaket eine positive Ladung erhält. Diese wird durch die Anionen ausgeglichen, die sich zusammen mit zeolithischen Kristallwasser in den Zwischenschichten befinden. Der Schichtaufbau wird in dem Röntgenpulverdiagramm deutlich (ASTM-Karte Nr.14-191), das zur Charakterisierung herangezogen werden kann.

Es sind auch synthetisch hergestellte Hydrotalcite bekannt, die z.B. in den DE-C 1 592 126, DE-A 3 346 943, DE-A 3 306 822 und EP-A 0 207 811 beschrieben sind.

In natürlichen und synthetischen Produkten kann das $Mg^{2+}$:$Al^{3+}$-Verhältnis zwischen etwa 1 und 5 variieren. Auch das Verhältnis von $OH^-$:$CO_3^{2-}$ kann schwanken. Natürliche und synthetische Hydrotalcite können durch die allgemeine Formel I

$$Mg_xAl(OH)_y(CO_3)_z . n\,H_2O \qquad (I)$$

näherungsweise beschrieben werden, wobei die Bedingungen $1 < x < 5$, $y > z$, $(y + 0,5z) = 2x + 3$ und $0 < n < 10$ gelten. Unterschiede in der Zusammensetzung der Hydrotalcite, insbesondere bezüglich des Wassergehaltes, führen zu Linienverschiebungen im Röntgenbeugungsdiagramm.

Natürliche oder synthetische Hydrotalcite geben beim Erhitzen bzw. Calcinieren kontinuierlich Wasser ab. Die Entwässerung ist bei 200°C vollständig, wobei durch Röntgenbeugung nachgewiesen werden konnte, daß die Struktur des Hydrotalcits noch erhalten geblieben ist. Die weitere Temperaturerhöhung führt unter Abspaltung von Hydroxylgruppen (als Wasser) und von Kohlendioxid zum Abbau der Struktur. Natürliche und nach verschiedenen Verfahren, z.B. gemäß den obigen Veröffentlichungen, künstlich hergestellte Hydrotalcite zeigen bei der Calcinierung ein generell ähnliches Verhalten.

Calcinierte Hydrotalcite sind bereits für verschiedene Zwecke eingesetzt worden, z.B. als Absorptionsmittel sowie bei Umsetzungen von Alkylenoxiden mit Alkylacetaten zur Herstellung von Mono-, Di- und Triethylenglykolethylether-acetaten, vgl. JP-A 56/36 431, referiert in C.A.95(11)97 099m (1981).

Unter Verbindungen mit aktiven H-Atomen im Sinne der Erfindung sind z.B. Fettalkohole, Fettsäuren und Amine zu verstehen, die bei der Ethoxylierung bzw. Propoxylierung nichtionische Detergentien bilden. Ein typisches Beispiel hierfür ist die Umsetzung von Fettalkoholen mit üblicherweise 10 bis 18 Kohlenstoffatomen mit Ethylenoxid und/oder Propylenoxid in Gegenwart von Katalysatoren, wobei die Fettalkohole mit mehreren Molekülen Ethylenoxid und/oder Propylenoxid reagieren.

Als Katalysatoren für die vorgenannte Polyalkoxylierung sind hierfür u.a. die folgenden eingesetzt worden:
Calcium- und Strontiumhydroxide, -alkoxide und phenoxide (EP-A 00 92 256),
Calciumalkoxide (EP-A 00 91 146),
Bariumhydroxid (EP-B 0 115 083),
basische Magnesiumverbindungen, z.B. Alkoxide (EP-A 00 82 569),
Magnesium- und Calciumfettsäuresalze (EP-A 0 85 167).

Die vorgenannten Katalysatoren weisen u.a. den Nachteil auf, daß sie schlecht in das Reaktionssystem einarbeitbar und/oder schwierig herstellbar sind.

Gebräuchliche Polyalkoxylierungskatalysatoren sind weiterhin Kaliumhydroxid und Natriummethylat.

Für Fettalkoholpolyalkoxylate ist eine enge Bandbreite des Polyalkoxylierungsgrades von besonderer Bedeutung, vgl. JAOCS, Vol. 63, 691 - 695 (1986), und HAPPI, 52 - 54 (1986). Die sogenannten "narrow-range"-Alkoxylate weisen demnach insbesondere die folgenden Vorteile auf:

- niedrige Fließpunkte
- höhere Rauchpunkte
- weniger Mole Alkoxid zum Erreichen der Wasserlöslichkeit
- weniger Hydrotope für das Einbringen in flüssige Universalwaschmittel
- ein geringerer, durch Anwesenheit freier (nicht umgesetzter) Fettalkohole bedingter Geruch

- Reduzierung des Plumings beim Sprühtrocknen von Waschmittelslurries, die Fettalkoholpolyalkoxylat-Tenside enthalten.

Es wurde nun gefunden, daß man unter erfindungsgemäßer Verwendung calcinierter Hydrotalcite als Katalysatoren Verbindungen mit aktiven H-Atomen bei kurzen Reaktionszeiten mit hohen Ausbeuten polyalkoxylieren kann und die Reaktionsprodukte mit einer engen Bandbreite bzw. Homologenverteilung erhalten werden können, wobei die Verteilungskurve der nach Poisson berechneten sehr nahe kommt.

Für die Zwecke der Erfindung eignen sich sämtliche der durch Calcinierung aus den eingangs genannten natürlichen und/oder synthetischen Hydrotalcite erhältlichen Katalysatoren; bevorzugt sind Hydrotalcite, die vor der Calcinierung die allgemeine Formel I

$$Mg_xAl(OH)_y(CO_3)_z \cdot n\,H_2O \qquad (I)$$

mit den oben angegebenen Bedingungen für x, y, z und n aufweisen; besonders bevorzugt sind Werte für x von 1,8 bis 3.

Die erfindungsgemäß eingesetzten calcinierten Hydrotalcite weisen den Vorteil auf, daß sie in das Reaktionsgemisch der Alkoxylierung leicht eingearbeitet werden und wegen ihrer Unlöslichkeit in dem Reaktionsgemisch durch einfache Maßnahmen wieder abgetrennt werden können. Sie können jedoch auch in den Reaktionsgemisch verbleiben, wenn ihre Anwesenheit bei der Weiterverwendung der Reaktionsprodukte nicht stört.

Gemäß einer vorteilhaften Ausführungsform der Erfindung sind die Verbindungen mit aktiven H-Atomen aus der von Fettsäuren, Hydroxyfettsäuren, Fettsäureamiden, Alkanolen, Alkylphenolen, Polyglykolen, Fettaminen, Fettsäurealkanolamiden oder vicinal hydroxy, alkoxy-substituierten Alkanen gebildeten Gruppe ausgewählt.

Beispiele für erfindungsgemäß unter Verwendung von calcinierten Hydrotalciten alkoxylierbare Verbindungen sind im folgenden aufgeführt.

Fettsäuren:

Fettsäuren mit 8 bis 22 Kohlenstoffatomen natürlicher oder synthetischer Herkunft, insbesondere geradkettige, gesättigte oder ungesättigte Fettsäuren einschließlich technischer Gemische derselben, wie sie durch Fettspaltung aus tierischen und/oder pflanzlichen Fetten und Ölen zugänglich sind, z.B. aus Kokosöl, Palmkernöl, Palmöl, Soyaöl, Sonnenblumenöl, Rüböl, Baumwollsaatöl, Fischöl, Rindertalg und Schweineschmalz; spezielle Beispiele sind Capryl-, Caprin-, Laurin-, Laurolein-, Myristin-, Myristolein-, Palmitin-, Palmitolein-, Öl-, Elaidin-, Arachin-, Gadolein-, Behen-, Brassidin- und Erucasäure; weiterhin methylverzweigte, gesättigte und ungesättigte Fettsäuren mit 10 bis 22 Kohlenstoffatomen, die bei der Dimerisierung von den entsprechenden ungesättigten Fettsäuren als Nebenprodukte entstehen, und Monocarbonsäuren mit 1 bis 7 Kohlenstoffatomen.

Hydroxyfettsäuren:

Natürliche oder synthetische Hydroxyfettsäuren, insbesondere mit 16 bis 22 Kohlenstoffatomen, z.B. Ricinolsäure oder 12-Hydroxystearinsäure.

Fettsäureamide:

Derivate der oben genannten geradkettigen, gesättigten oder ungesättigten Fettsäuren mit Ammoniak oder primären aliphatischen Aminen mit 1 bis 4 Kohlenstoffatomen in dem aliphatischen Substituenten.

Alkanole:

Gesättigte oder ungesättigte Monoalkanole, insbesondere Hydrierungsprodukte der oben genannten geradkettigen, gesättigten oder ungesättigten Fettsäuren bzw. Derivate derselben wie Methylester oder Glyceride; aliphatische oder cyclische Alkanole mit 2 bis 6 Kohlenstoffatomen, z.B. Ethanol, Propanol, Butanol, Hexanol und Cyclohexanol; einschließlich der von den vorgenannten Monoalkanolen abgeleiteten Guerbet-Alkohole.

3

Alkylphenole:

Mono-, Di- oder Trialkylphenole, insbesondere mit 4 bis 12 Kohlenstoffatomen in den Alkylgruppen.

Polyglykole:

Polyethylen- oder Polypropylenglykole (durchschnittlicher Polymerisationsgrad 2 bis 2000).

Fettamine:

Insbesondere primäre Fettamine, die aus Nitrilen der oben genannten geradkettigen, gesättigten oder ungesättigten Fettsäuren oder den entsprechenden Fettalkoholen zugänglich sind; weiterhin auch Mono- und Dialkylamine mit $C_1$-$C_6$-Alkylgruppen.

Fettsäurealkanolamide:

Derivate der oben genannten geradkettigen, gesättigten oder ungesättigten Fettsäuren mit Mono- oder Dialkanolaminen, insbesondere Mono- oder Diethanolamin.

Vicinal hydroxy, alkoxy-substituierte Alkane:

Ringöffnungsprodukte von 1,2-Epoxyalkangemischen mit 12 bis 22 Kohlenstoffatomen in der Kette mit mehrwertigen Alkanolen mit 2 bis 12 Kohlenstoffatomen und 2 bis 6 Hydroxylgruppen.

Die erfindungsgemäß unter Verwendung von calcinierten Hydrotalciten herzustellenden Derivate sind handelsübliche Produkte, so daß sich eine nähere Erläuterung erübrigt. Sie werden durchweg durch Ethoxylierung und/oder Propoxylierung aktive Wasserstoffatome aufweisender Ausgangsverbindungen hergestellt. Typische Vertreter sind beispielsweise ein Anlagerungsprodukt von 9 Mol Ethylenoxid an Kokosölfettsäure, ein Anlagerungsprodukt von 2 Mol Ethylenoxid an ein Fettalkoholgemisch der Kettenlänge C12-14, ein Anlagerungsprodukt von 3 Mol Ethylenoxid und 8 Mol Propylenoxid an ein Fettalkoholgemisch der Kettenlänge C12-18, ein Anlagerungsprodukt von 10 Mol Ethylenoxid an Nonylphenol, ein Anlagerungsprodukt von 7,3 Mol Ethylenoxid an Glycerin, ein Anlagerungsprodukt von 10 Mol Ethylenoxid an ein Diolgemisch, das durch Umsetzung eines 1,2-Epoxyalkangemisches der Kettenlänge C12-16 mit Ethylenglykol erhalten wurde, ein Anlagerungsprodukt von 12 Mol Ethylenoxid an ein Fettamingemisch der Kettenlänge C10-18 und ein Anlagerungsprodukt von 4 Mol Ethylenoxid an Kokosfettsäuremonoethanolamid.

Gemäß einer weiteren vorteilhaften Ausführungform der Erfindung setzt man die calcinierten Hydrotalcite in einer Menge von 0,1 bis 2 Gew.-%, bezogen auf das Endprodukt der Ethoxylierung bzw. Propoxylierung, den Reaktionsgemischen zu.

Die erfindungsgemäß einzusetzenden calcinierten Hydrotalcite können aus den natürlichen oder synthetischen Hydrotalciten durch mehrstündiges Erhitzen auf Temperaturen von über 100°C erhalten werden; besonders bevorzugt sind Calcinierungstemperaturen von 400 bis 600°C.

Die Erfindung wird im folgenden anhand bevorzugter Ausführungsbeispiele näher erläutert, wobei auf die Zeichnungen Bezug genommen wird. Die Zeichnungen zeigen die gemäß einigen Beispielen erzielten Homologenverteilungen im Vergleich zu den bei der Verwendung von Natriummethylat erzielbaren.

Beispiel 1.

Ein handelsüblicher synthetischer Hydrotalcit wurde 8 h bei 500°C calciniert.

Zur Umsetzung eines handelsüblichen Laurylalkohols mit 6 mol Ethylenoxid wurde der Laurylalkohol in einem Druckreaktor vorgelegt und mit 0,5 Gew.-%, bezogen auf erwartetes Endprodukt, des zuvor erhaltenen calcinierten Hydrotalcits versetzt. Der Reaktor wurde mit Stickstoff gespült und 30 min lang bei einer Temperatur von 100°C evakuiert. Anschließend wurde die Temperatur auf 180°C gesteigert und die gewünschte Menge Ethylenoxid bei einem Druck von 4 bis 5 bar aufgedrückt. Nach Beendigung der Reaktion ließ man 3o min. nachreagieren. Nach dem Abfiltrieren von suspendiertem Katalysator erhielt man das gewünschte Reaktionsgemisch, dessen Kenndaten aus Tabelle 1 ersichtlich sind.

4

Beispiele 2 bis 13.

Analog zu der in Beispiel 1 beschriebenen Weise wurden die in Tabelle 1 aufgeführten, aktive H-Atome aufweisenden Verbindungen unter Verwendung calcinierter synthetischer Hydrotalcite mit Ethylenoxid umgesetzt. Die eingesetzten Verbindungen, die umgesetzten Mengen Ethylenoxid, die Calcinierungsbedingungen für die Hydrotalcite, die Katalysatorkonzentration, die Reaktionszeit der Ethoxylierung sowie die OH-Zahlen einiger der erhaltenen Ethoxylierungsprodukte sind in Tabelle 1 für einige Verbindungen zusammengefaßt. Weiterhin ist in der Tabelle 1 für einige Verbindungen vermerkt, in welcher Zeichnung die erzielten Homologenverteilungen im Vergleich zu Natriumethylat wiedergegeben sind.

In den Beispielen 1, 2, 6 und 7 wurden calcinierte Hydrotalcite eingesetzt, bei denen das Atomverhältnis von Mg zu Al (entsprechend zu x in der obigen allgemeinen Formel) 2,17 betrug. Für die calcinierten Hydrotalcite der Beispiele 3, 4 und 8 bis 13 betrug das Mg/Al-Atomverhältnis 2,17, für den des Beispiels 5 2,08.

EP 0 339 426 B1

## Tabelle 1

### Ethoxylierung von H-reaktiven Verbindungen

| Bsp. Nr. | Produkt | Hydrotalcit (Calcinier- ungsbedin- gungen) | Katalysator- konz. | Reaktions- zeit | Produkt-OHZ[1] ist | soll | Homologen- verteilung |
|---|---|---|---|---|---|---|---|
| 1 | $C_{12}$ + 6EO | 8 h/500°C | 0,5 | 8 | 135,4 | 125 | Fig.1 |
| 2 | $C_{12/14}$ + 2EO | 8 h/500°C | 0,5 | 3 | 198,2 | 200 | Fig.2 |
| 3 | $C_{12}$ + 6EO | 4 h/500°C | 0,5 | 7,5 | 132,1 | 135,7 | Fig.3 |
| 4 | $C_{12/14}$ + 2EO | 4 h/500°C | 0,5 | 2 | 201,8 | 200 | Fig.4 |
| 5 | $C_{12}$ + EO | 4 h/500°C | 0,5 | 7,5 | 128,3 | 125 | Fig.5 |
| 6 | $C_{18'}/18^2$ + 4,6EO | 8 h/500°C | 0,5 | 8,5 | 123,6 | 117,8 | Fig.6 |
| 7 | $C_8$ + 4EO | 8 h/500°C | 0,5 | 9,0 | 196,3 | 184,8 | Fig.7 |
| 8 | $C_{12}$ + 6EO | 4 h/600°C | 0,5 | 6,5 | 130,9 | 131,7 | Fig.8 |
| 9 | 2-Octyldodecanol + 2EO | 4 h/500°C | 0,5 | 0,75 | 140,7 | 145,1 | |
| 10 | Cyclohexanol x 4EO | 4 h/500°C | 0,5 | 2 | 274,9 | 223,5 | |
| 11 | Butanol x 10EO x 10PO | 4 h/500°C | 0,5 | 3,5 | 55,3 | 51,4 | |
| 12 | Laurinsäure x 6EO | 4 h/500°C | 0,5 | 3,5 | 143,4 | 132,3 | |
| 13 | Kokosamin x 12EO | 4 h/500°C | 0,5 | 4 | 244,7 | 219,4 | |

1) OHZ = Hydroxylzahl

2) Technisches Oleylalkohol($C_{18'}$)/Stearylalkohol($C_{18}$)-Gemisch

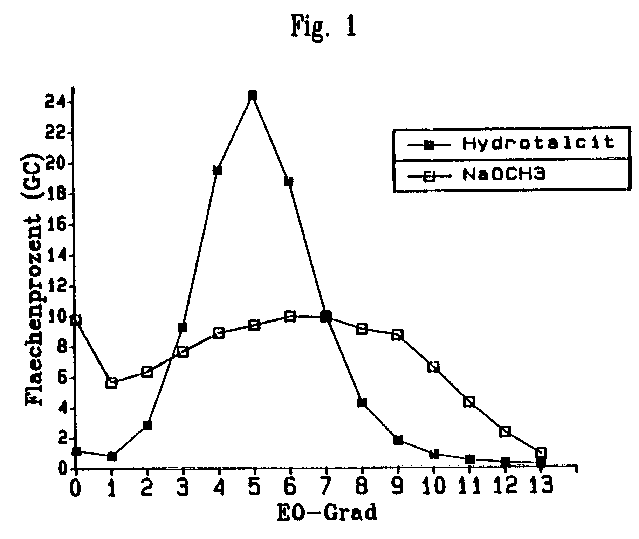

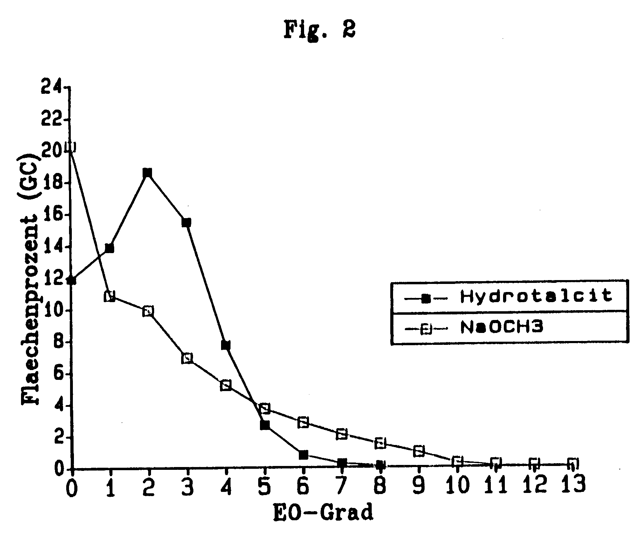

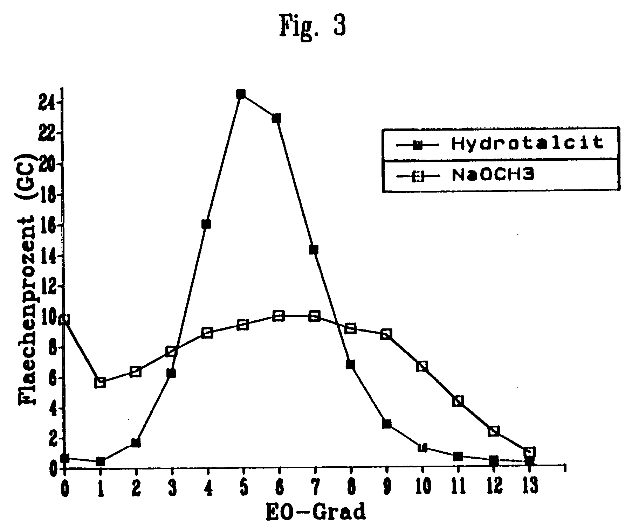

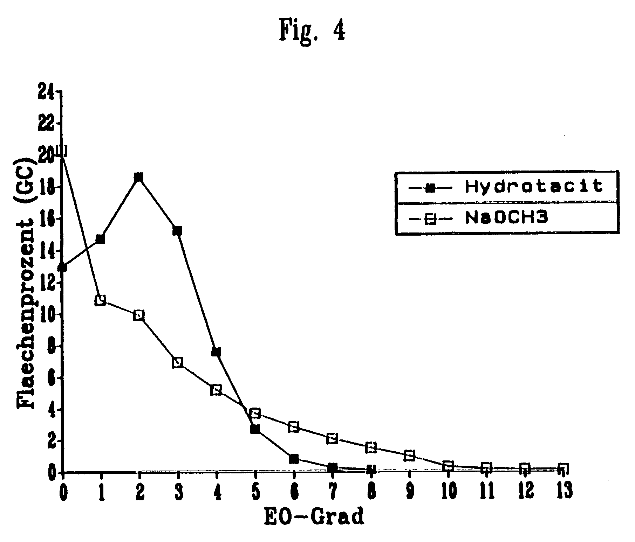

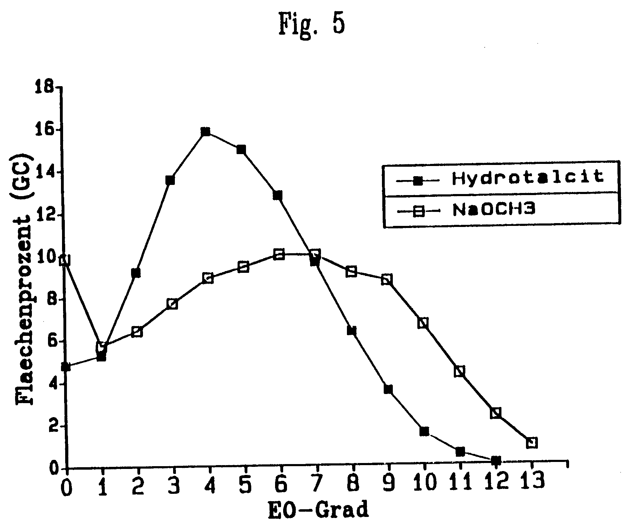

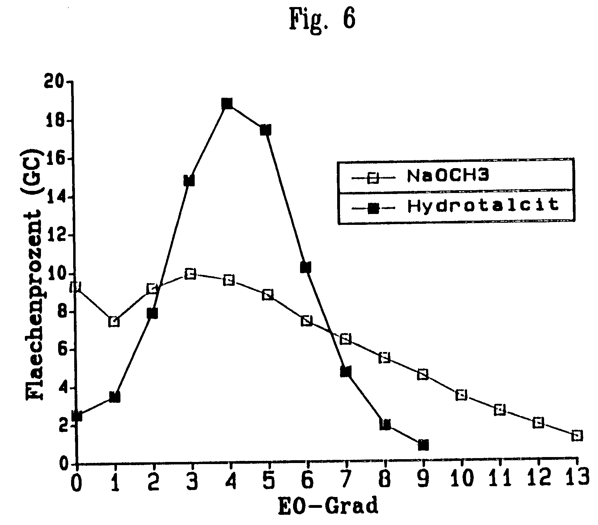

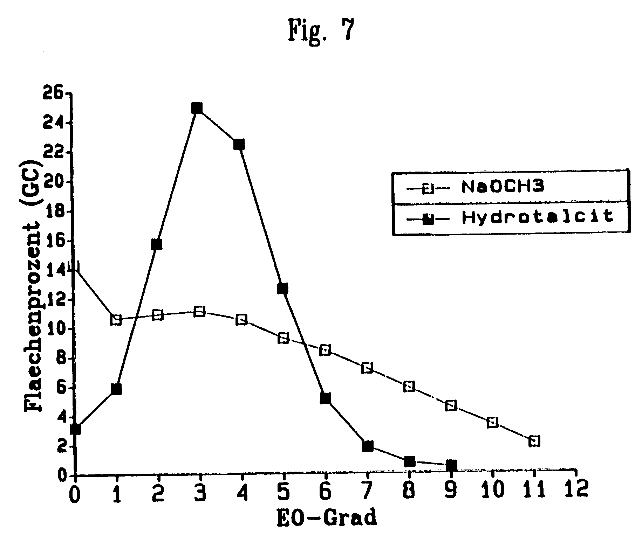

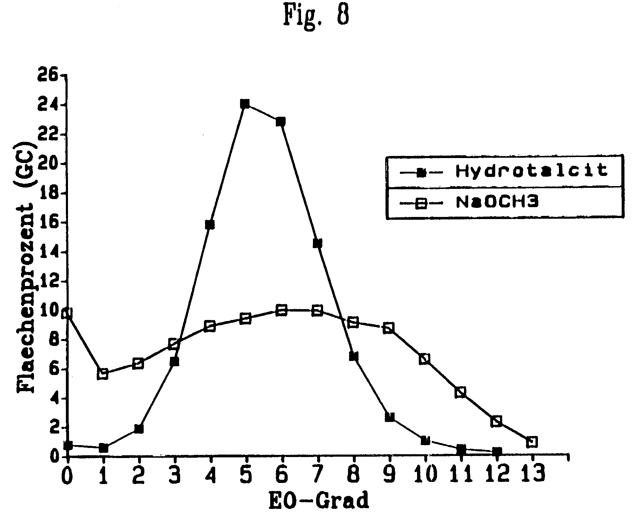

## Patentansprüche

1. Verwendung von durch mehrstündiges Erhitzen auf Temperaturen von über 100 °C calcinierten Hydrotalciten als Katalysatoren für die Ethoxylierung bzw. Propoxylierung von Verbindungen mit aktiven H-Atomen.

2. Verwendung nach Anspruch 1, dadurch gekennzeichnet, daß die Verbindungen mit aktiven H-Atomen aus der von Fettsäuren, Hydroxyfettsäuren, Fettsäureamiden, Alkanolen, Alkylphenolen, Polyglykolen, Fettaminen, Fettsäurealkanolamiden oder vicinal hydroxy, alkoxysubstituierten Alkanen gebildeten Gruppe ausgewählt sind.

3. Verwendung nach Anspruch 1 oder 2, dadurch gekennzeichnet, daß die Hydrotalcite vor dem Calcinieren eine Zusammensetzung der Formel I

$$Mg_xAl(OH)_y(CO_3)_z \cdot n\,H_2O \qquad (I)$$

aufweisen, in der die Bedingungen $1 < x < 5$, $y > z$, $(y + 0,5\,z) = 2x + 3$ und $0 < n < 10$ gelten.

4. Verwendung nach mindestens einem der Ansprüche 1 bis 3, dadurch gekennzeichnet, daß für die Hydrotalcite der allgemeinen Formel I

$$Mg_xAl(OH)_y(CO_3)_z \cdot n\,H_2O$$

x eine Zahl von 1,8 bis 3 ist und y, z sowie n wie oben definiert sind.

5. Verwendung nach mindestens einem der Ansprüche 1 bis 4, dadurch gekennzeichnet, daß die Hydrotalcite bei Temperaturen zwischen 400 und 600 °C calciniert wurden.

6. Verwendung nach mindestens einem der Ansprüche 1 bis 5, dadurch gekennzeichnet daß man die calcinierten Hydrotalcite in einer Menge von 0,1 bis 2 Gew.-%, bezogen auf das Endprodukt der Ethoxylierung bzw. Propoxylierung, einsetzt.

**Claims**

1. The use of hydrotalcites calcined by heating for several hours at temperatures above 100 °C as catalysts for the ethoxylation or propoxylation of compounds containing active H atoms.

2. The use claimed in claim 1, characterized in that the compounds containing active H atoms are selected from the group consisting of fatty acids, hydroxyfatty acids, fatty acid amides, alkanols, alkylphenols, polyglycols, fatty amines, fatty acid alkanolamides or vicinally hydroxy, alkoxy-substituted alkanes.

3. The use claimed in claim 1 or 2, characterized in that, before calcination, the hydrotalcites have a composition corresponding to formula I:

$$Mg_xAl(OH)_y(CO_3)_z \cdot n\,H_2O \qquad (I)$$

in which the conditions $1 < x < 5$, $y > z$, $(y + 0.5\,z) = 2x + 3$ and $0 < n < 10$ apply.

4. The use claimed in at least one of claims 1 to 3, characterized in that, for the hydrotalcites corresponding to general formula I:

$$Mg_xAl(OH)_y(CO_3)_z \cdot n\,H_2O$$

x is a number of 1.8 to 3 and y, z and n are as defined above.

5. The use claimed in at least one of claims 1 to 4, characterized in that the hydrotalcites are calcined at temperatures of 400 to 600 °C.

6. The use claimed in at least one of claims 1 to 5, characterized in that the calcined hydrotalcites are used in a quantity of 0.1 to 2% by weight, based on the end product of the ethoxylation or propoxylation reaction.

**Revendications**

1. Utilisation d'hydrotalcites, calcinées par chauffage pendant plusieurs heures à des températures supérieures à 100 °C, comme catalyseurs pour l'éthoxylation ou la propoxylation de composés comportant des atomes d'hydrogène actifs.

2. Utilisation selon la revendication 1, caractérisée en ce que les composés comportant des atomes d'hydrogène actifs sont sélectionnés parmi le groupe constitué des acides gras, hydroxyacides gras, amides d'acides gras, alcanols, alkylphénols, polyglycols, amines grasses, alcanolamides d'acides gras ou alcanes à substitution vicinale hydroxylique, alcoxylique.

3. Utilisation selon la revendication 1 ou 2, caractérisée en ce que les hydrotalcites présentent avant la calcination une composition de formule I

$Mg_xAl(OH)_y(CO_3)_z . n H_2O$     (I)

dans laquelle, les conditions $1 < x < 5$, $y > z$, $(y + 0,5 z) = 2 x + 3$ et $0 < n < 10$ sont d'application.

4. Utilisation selon au moins l'une des revendications 1 à 3, caractérisée en ce que pour les hydrotalcites de la formule générale I

$Mg_xAl(OH)_y(CO_3)_z . n H_2O$

x est un nombre de 1,8 à 3 et y, z et n possèdent la valeur indiquée ci-avant.

5. Utilisation selon au moins l'une des revendications 1 à 4, caractérisée en ce que les hydrotalcites sont calcinées à des températures comprises entre 400 et 600 °C.

6. Utilisation selon au moins l'une des revendications 1 à 5, caractérisée en ce que l'on met en oeuvre les hydrotalcites calcinées en proportions de 0,1 à 2 % en poids par rapport au produit final de l'éthoxylation ou de la propoxylation.

Fig. 1

Fig. 2

Fig. 3

EP 0 339 426 B1

Fig. 4

Fig. 5

EP 0 339 426 B1

Fig. 6

# Fig. 7

EP 0 339 426 B1

Fig. 8